# EUROPÄISCHE PATENTSCHRIFT

(11) **EP 1 924 224 B1**
(45) Veröffentlichungstag und Bekanntmachung des Hinweises auf die Patenterteilung: **13.05.2015**
(21) Anmeldenummer: 06805720.7
(22) Anmeldetag: 14.09.2006
(51) Int. Cl.: A61F 2/24

(54) **MRT KOMPATIBLE KLAPPENPROTHESE ZUR VERWENDUNG IM MENSCHLICHEN ODER TIERISCHEN KÖRPER ZUM ERSATZ EINER ORGANKLAPPE ODER GEFÄSSKLAPPE**
MRT-COMPATIBLE VALVE PROSTHESIS FOR USE IN THE HUMAN OR ANIMAL BODY FOR REPLACEMENT OF AN ORGAN VALVE OR A VESSEL VALVE
PROTHESE VALVULAIRE A COMPATIBILITE MRT A UTILISER DANS L'ORGANISME HUMAIN OU ANIMAL POUR REMPLACER UNE VALVULE D'ORGANE OU DE VAISSEAU

(30) Priorität: 14.09.2005 DE 102005044009
(43) Veröffentlichungstag der Anmeldung: 28.05.2008
(60) Teilanmeldung: 12005881.3
(73) Patentinhaber: Vueklar Cardiovascular Ltd., Glentirranmuir Kippen Stirling FK8 3HU (GB)
(72) Erfinder: MELZER, Andreas, 45481 Mülheim/Ruhr (DE); IMMEL, Erwin, 45897 Gelsenkirchen (DE)
(74) Vertreter: Von Rohr Patentanwälte Partnerschaft mbB
(86) Internationale Anmeldenummer: PCT/EP2006/008964
(87) Internationale Veröffentlichungsnummer: WO 2007/031316

(56) Entgegenhaltungen:
- EP-A2- 1 092 985
- US-A1- 2005 049 692
- US-B1- 6 767 360

## Beschreibung

Die Erfindung betrifft eine biologische oder künstliche Klappenprothese zur Verwendung im menschlichen oder tierischen Körper zum Ersatz einer Organklappe oder Gefäßklappe, insbesondere Herz- oder Venenklappenprothese, mit einem Stent oder stentlos, mit einem tragenden Klappengerüst, mit wenigstens einer Klappe und mit mindestens einer Leiterschleife, die die Induktivität eines elektrischen Resonanzschwingkreises ausbildet.

Venenklappen befinden sich biologisch in den tiefen Beinvenen. Sie dienen der Verhinderung des Rückflusses des venösen Blutes in die Gliedmaßen. Durch Kompression der Venen beim Anspannen der Muskeln wird das Blut in Richtung des Herzens transportiert. Die Klappen können undicht (insuffizient) werden oder ihre Funktion durch Anlagerung von geronnenem Blut (Beinvenenthrombose) verlieren. Venenklappenprothesen sind derzeit in der Entwicklung und bestehen in der Regel aus einem Stentgerüst aus selbstexandierendem Material, wie beispielsweise Nitinol oder einem Stahlgerüst, in welches entweder eine Klappe aus Polymer oder ein Dünnfilm aus Nitinol oder ein Metallgeflecht eingearbeitet sind. Die Klappenfunktion kann ggf. durch die Anlage einer Gewebemanschette mit Einengung der Vene chirurgisch wiederhergestellt werden.

Die Herzklappen befinden sich innerhalb des Herzens und trennen die Vorhöfe von den Herzkammern, wobei sie als Rückschlagventil dafür sorgen, daß das Blut in die richtige Richtung fließt. Das Herz hat vier Klappen, wovon jeweils zwei dem rechten und dem linken Herzen angehören. Das Blut gelangt aus den venösen Gefäßen in den Vorhof oder die Vorkammer, von dort über eine Klappe in die Herzkammer und von dort wiederum über eine Klappe in die Gefäße des großen oder kleinen Kreislaufs. Die Herzklappen bestehen aus dünnen, stabilen Bindegewebsschichten, die taschenförmig (Aortenklappe und Pulmonalklappe) bzw. segelförmig (Mitralklappe und Trikuspidalklappe) geformt sind. Sie sind wie der übrige Herzinnenraum mit der glatten Innenhaut des Herzens (Endokard) überzogen,

Bei erheblicher Einschränkung der Klappenfunktion durch angeborene oder erworbene Defekte, wie Klappeninsuffizienz oder Klappenstenose, ist der Ersatz der Herzklappe durch eine Prothese erforderlich. Es gibt zwei Arten von Herzklappenprothesen, nämlich die biologische und die mechanische Herzklappe. Die biologischen Herzklappen werden auch stentlose Klappenprothesen genannt, da sie nicht über ein stabilisierendes Gerüst verfügen. Die Klappen sind präparierte Schweineherzklappen oder werden aus dem Gewebe des Rinderherzbeutels oder der Submusosa des Schweinedünndarms hergestellt oder aus menschlichen Endothelzellen auf Kollagenfasern oder Kunststoff-, Metallnetzen/-folien entsprechend der Form und Funktion der menschlichen Herzklappe gezüchtet. Diese Prothesen machen in den meisten Fällen keine lebenslange orale Antikoagulation zur Blutverdünnung notwendig, sondern nur eine temporäre Gerinnungshemmung in den ersten Monaten nach der Operation.

Mechanische Klappen bestehen aus Metall und/oder Kunststoff. Sie erfordern eine lebenslange Antikoagulation, sind aber in den meisten Fällen nahezu unbegrenzt haltbar. Die Klappen sind von einem Gewebegeflecht umhüllt, mit dem diese in das Lager der ehemaligen defekten Herzklappe eingenäht werden.

Die Operationen zum Ersatz defekter Herzklappen mit Prothesen werden in der Herzchirurgie unter Herbeiführung eines (kardioplegischen, diastolischen) Herzstillstandes durchgeführt, um einen blutfreien, ruhigen Operationsitus zu erhalten. In diesem Zustand werden verschiedenste Arten von Herzklappen eingenäht. Als Ausgleich zu diesem Herzstillstand muß zum einen eine Herzlungenmaschine den Kreislauf aufrechterhalten, zum anderen muß die Hauptschlagader (Aorta) abgeklemmt werden. Diese Maßnahmen sind mit erheblichen Nebenwirkungen und Komplikationen verbunden.

Die aktuellen Entwicklungen gehen daher in die Richtung einer Implantation von faltbaren Herzklappenprothesen, die über Katheter eingeführt und plaziert werden. Diese Prothesen basieren auf einem stentartigen Gerüst, in das entweder eine biologische Klappe oder eine Polymer-Klappe eingebaut ist. Die Implantation erfolgt durch Ballondilatation oder selbstexpandierend. Von Nachteil ist jedoch, daß die Stentgerüste der faltbaren, bekannten Herzklappen-Prothesen eine MRT-Visualisierung und damit eine sehr einfache Überprüfung der Klappenfunktion beeinträchtigen.

Eine perkutan implantierbare Herzklappenprothese kann das Risiko, das bei einer offenen Operation vorhanden ist, deutlich minimieren, jedoch ist die präzise Positionierung unter Röntgenkontrolle nicht möglich, da das Weichgewebe des Herzens nicht dargestellt wird und nur mittels Kontrastmittelgabe der blutdurchflossene Raum erscheint. Eine Implantation durch MRT-Bildgebung wäre vorteilhaft, da sowohl das strömende Blut als auch das Herzgewebe ohne Kontrastmittel dargestellt werden.

Die derzeit am Markt verfügbaren faltbaren Herzklappen haben nach dem heutigen Stand der Technik noch keine optimalen Eigenschaften, denn die Funktion der Implantate kann nur eingeschränkt, invasiv durch den Einsatz von Kathetern, Röntgenstrahlung und Kontrastmitteln mit erheblichen Risiken und Belastungen für die Patienten und das medizinische Personal kontrolliert werden. Sowohl beim Setzen des Implantats als auch bei den erforderlichen Nachuntersuchungen sind häufig lange Durchleuchtungszeiten erforderlich. Diese betragen zwischen 10 und 15 Minuten, können aber auch durchaus 60 Minuten und mehr umfassen. Aus den langen Expositionszeiten resultieren Nebenwirkungen wie die Entwicklung von Strahlengeschwüren (Ulceration), die zu Hautkrebs führen können. Die langen Durchleuchtungszeiten und entsprechend hohe Dosiswerte haben auch dazu geführt, daß Patienten, die sich einer kardiologischen Intervention unterzogen haben, Strahlenschäden erlitten haben. Das Bundesamt für Strahlenschutz hat diesbezüglich in einer Untersuchung festgestellt, daß die Reduktion der Strahlenexposition in der medizinischen Diagnostik von besonderer Bedeutung ist.

Untersuchungstechniken der Magnet-Resonanz-Tomographie (MRT) können zur Verminderung röntgendiagnostischer und nuklearmedizinischer Untersuchungsverfahren erheblich beitragen. Neben der fehlenden Strahlenbelastung hat die Magnet-Resonanz-Tomographie im Vergleich zu röntgentechnischen Verfahren wie der Computertomographie und der Durchleuchtung weitere Vorteile. Sie bietet einen hervorragenden Weichteilkontrast und als einziges aller Schichtbildverfahren die völlig freie Schichtpositionierung. Ortsaufgelöste spektrometrische Techniken erlauben eine funktionelle Analyse biochemischer Vorgänge im menschlichen Körper. Ebenso können Gefäße ohne Verwendung von Kontrastmitteln direkt im Operationsgebiet dargestellt werden. Im offenen Magnet-Resonanz-Tomographen hat der Arzt Zugang zum Patienten und kann so unter direkter MRT-Kontrolle Punktionen und Operationen vornehmen. Katheterisierungen sind auch in geschlossenen Hochfeldgeräten mit moderner Bildgebung gut durchzuführen.

Eine Herzklappenprothese der eingangs genannten Art ist bereits aus der EP 1 092 985 A2 bekannt, aus der auch das MR-Bildgebungsverfahren hervorgeht, worauf ausdrücklich Bezug genommen wird. Die bekannte Herzklappenprothese weist ein äußeres Klappengerüst auf, an dem die eigentliche Herzklappe beweglich gelagert ist., Zur Ausbildung des Resonanzschwingkreises sind sowohl Spulen als auch Kondensatoren am ringförmigen Traggerüst vorgesehen. Vorteilhaft bei der bekannten Herzklappenprothese ist, daß sich diese hervorragend durch das MR-Bildgebungsverfahren darstellen läßt. Von Nachteil ist, daß diese Prothese nicht perkutan implantierbar ist.

Konventionelle faltbare Herzklappen lassen sich zwar perkutan implantieren, die Darstellung im Magnet-Resonanz-Tomographen hat gegenwärtig jedoch folgende entscheidende Nachteile:
- eine starke Störungs- und Artefaktempfindlichkeit aufgrund der verwendeten Metalle,
- das Innere eines Metall-Implantats ist aufgrund des Faraday-Käfigeffektes abgeschirmt und nur schwer sichtbar,
- metall- oder kunststoffbasierte Klappenmechanismen sind entweder nicht signalgebend oder führen zu Magnetfeldinhomogenisierung,
- in bewegten Metallkomponenten des Klappenmechanismus im starken Magnetfeld können unerwünschte induktive Effekte auftreten,
- die biologischen Klappen bestehen überwiegend aus nicht- bis schwachsignalgebenden kollagenen Fasern und wenigen Zellen, so daß eine direkte MRT-Bildgebung erschwert ist,
- die zeitliche und örtliche Auflösung zur vollständigen Diagnostik der Klappe und ihrer Funktionen sind unzureichend.

Aufgrund der vorstehenden Probleme sind konventionelle faltbare Herzklappen, die üblicherweise aus Edelstahllegierungen oder Nitinol bestehen, für MRT-Untersuchungen der Klappenfunktionen und der korrekten Lagen der Prothese nicht oder nur unzureichend einsetzbar.

Aufgabe der vorliegenden Erfindung gemäß Anspruch 1 ist es daher, eine Klappenprothese der eingangs genannten Art zur Verfügung zu stellen, bei der die zuvor beschriebenen Nachteile vermieden werden.

Die vorgenannte Aufgabe ist bei einer Klappenprothese der eingangs genannten Art erfindungsgemäß dadurch gelöst, daß die wenigstens eine Leiterschleife das Klappengerüst selbst und/oder die Klappe selbst ausbildet. Die erfindungsgemäße Ausgestaltung bietet eine Reihe von erheblichen Vorteilen, wobei die wenigstens eine Leiterschleife eine Mehrzahl von Funktionen übernimmt. Zunächst einmal bildet die Leiterschleife den MR-Schwingkreis und stellt damit die MR-Funktion sicher. Darüber hinaus bildet die Leiterschleife das Klappengerüst. Außerdem kann die Leiterschleife auch die Klappe ausbilden. Da die Leiterschleife bei der erfindungsgemäßen Prothese eine tragende Funktion übernimmt, ist es über die Leiterschleife auch möglich, die Durchgangsöffnung der Prothese offenzuhalten. Schließlich ergibt sich insbesondere dann, wenn die Leiterschleife zusätzlich Verankerungsmittel aufweist, eine Verankerungs- bzw. Befestigungsfunktion.

Bevorzugt ist bei der Erfindung vorgesehen, daß der Stent und/oder das Klappengerüst außenseitig, das heißt an der äußeren umlaufenden Mantelfläche, Zellen unterschiedlicher Größe aufweist. Damit ergibt sich außenseitig eine Gitter- oder Netzform, wobei dann eine Vielzahl von gleichgroßen und auch unterschiedlich großen Zellen bzw. Öffnungen oder Fenstern vorgesehen sein können.

Darüber hinaus ist es dadurch, daß die Leiterschleife das Klappengerüst bildet, sehr einfach möglich, die Prothese insgesamt falt- bzw. expandierbar und damit perkutan implantierbar auszubilden.

Letztlich ergibt sich durch die Erfindung damit eine Klappenprothese aus mindestens einem geschlossenen Schwingkreis mit einer Induktivität und vorzugsweise einer Kapazität, die in zusammengefalteter Anordnung perkutan implantierbar ist, wobei eine optimale Stabilisierung und Verankerung der Prothese mittels der Schwingkreiskonstruktion und -geometrie erreicht wird. Der elektrische Schwingkreis erzeugt dabei eine Signalantwort, die mittels mindestens einer externen Empfangsspule detektiert und ortsaufgelöst dargestellt wird, so daß die MR-Bildgebung der Klappe und ihrer Funktion verbessert wird. Darüber hinaus ist die erfindungsgemäße Klappenprothese in besonderem Maße dazu geeignet, unter MR-Bildgebung zu implantieren, da die Entfaltung wie auch die Positionierung und Verankerung präzise bestimmt werden können.

Zur Verankerung der Leiterschleife bzw. des Schwingkreises bietet es sich an, Verankerungsmittel beispielsweise in Form von Ösen, Haken od. dgl. vorzusehen. Günstig ist es in diesem Zusammenhang, die Verankerungsmittel einstückig mit der Leiterschleife auszubilden, so daß zur Verankerung keine weiteren Bauteile erforderlich sind. Grundsätzlich ist es allerdings auch möglich, die Ösen, Haken od. dgl. als separate Bauteile auszubilden, die dann an dem Spulendraht nicht verschiebbar befestigt sind. Im Zusammenhang mit den Verankerungsmitteln ist von Bedeutung, daß diese im komprimierten Zustand der Klappenprothese nicht über diese überstehen und erst nach der Entfaltung, wenn sich die Klappenprothese an der Implantationsstelle befindet, wirksam werden und zum Befestigen der Prothese am umgebenden Gewebe genutzt werden können. Durch die Integration und unmittelbare Nähe der Verankerungen zum Schwingkreis ist insbesondere die korrekte Positionierung der Prothese unter MRT sowie die nachfolgende Diagnostik in bezug auf Undichtigkeit des Klappenlagers verbessert.

In besonderem Maße eignet sich die vorliegende Erfindung im Zusammenhang mit biologischen, stentlosen Klappenprothesen, insbesondere heterologe von einem Tier, wie von der Herzklappe oder dem Herzbeutel eines Schweins oder dem Herzbeutel eines Rinds, oder homologe und isologe von menschlichen Geweben und Zellen, wobei die Leiterschleife in diesen Fällen von der biologischen Herzklappen umschlossen ist. Letztlich beinhaltet in diesem Falle die biologische Herzklappe die Leiterschleife und damit den Schwingkreis. Dabei versteht es sich in diesem Zusammenhang, daß die Leiterschleife eine der natürlichen Herzklappe angepaßte Form aufweist, wobei die Klappenansätze oder -flügel vom Spulendraht in mindestens einer Windung umfaßt werden und die Basis der Herzklappe von einer Zylinderspule umschlossen ist.

Denn gerade in Verbindung mit einer biologischen Herzklappe bietet es sich an, daß bioresorbierbare Anteile, die die Klappenprothese für die Implantation stabilisieren und abdichten, vorgesehen sind. Dabei ist dann die wenigstens eine Leiterschleife zur Implantation in einem biologischen Anteil der Klappenprothese vorgesehen und verbleibt dort, nachdem die bioresorbierbaren Anteile im Körper abgebaut sind. Die bioresorbierbaren Anteile dienen damit lediglich der Stabilisation der Klappenprothese bis zum Zeitpunkt unmittelbar nach der Implantation. Die eingewachsene Herzklappe verfügt jedoch weiterhin über die vorteilhaften Eigenschaften für die MRT-Bildgebung, um langfristig auftretende Defekte wie Verschleiß, Gewebereaktion, optimal im MRT darzustellen und rechtzeitig eine Therapie oder einen Ersatz vornehmen zu können.

Während es grundsätzlich möglich ist, die Prothese ohne Stent auszubilden, versteht es sich, daß auch Prothesen mit Stent möglich sind. Hierbei ist dann erfindungsgemäß vorgesehen, daß das Klappengerüst und die Klappe in einem selbst entfaltbaren oder mechanisch, insbesondere hydraulisch bzw. fluidisch expandierbaren Stent durch die Leiterschleife zumindest teilweise derart fixiert sind, daß bei der Komprimierung und anschließenden Entfaltung sowohl das Klappengerüst und die Klappe als auch die Leiterschleife in die gewünschte Form, Position und Geometrie zurückkehren, wobei gleichzeitig die notwendige Abdichtung der Prothese im Lager erreicht werden kann. Besonders bevorzugt ist es in diesem Zusammenhang, daß der gesamte Klappenmechnismus über die Leiterschleife nicht nur gehalten ist, sondern daß die Leiterschleife gleichzeitig zur Befestigung der Klappe am Stent dient. Hierbei sind dann zumindest Bereiche der Leiterschleife aus dem Stent herausgeführt. Diese Bereiche können dabei auch der Verankerung im umgebenden Gewebe dienen und somit Verankerungsmittel darstellen.

Bevorzugt kann vorgesehen sein, daß das Gerüst mehrere Resonanzschwingkreise mit unterschiedlichen Resonanzfrequenzen mit jeweils einer Leiterschleife ausbildet oder daß mehrere Resonanzschwingkreise mit unterschiedlichen Resonanzfrequenzen mit nur einer Leiterschleife miteinander gekoppelt sind. Hierdurch wird nicht nur die Tragfunktion des Klappengerüstes verbessert, der Herzklappenersatz kann damit auch in unterschiedlichen MRTs eingesetzt werden. Bevorzugt ist es in diesem Zusammenhang, daß das Klappengerüst mehrere unterschiedliche Spulenformen, d. h. unterschiedliche Spulengeometrien wie beispielsweise Zylinder, Sattel- und Birdcagespulen aufweist.

Im übrigen kann mindestens eine Spule des Resonanzschwingkreises auch eine Mäanderstruktur aufweisen. Von Vorteil ist es im übrigen auch, wenn die Leiterschleife bzw. die Leiterbahn selbst mäanderförmig ausgebildet ist. Durch diese Ausbildung ergibt sich eine gezielte und definierte Faltbarkeit und Expansion, wobei die Fläche der Spule erhöht werden kann und die Elastizität im Lagergewebe verbessert wird. Gleichzeitig kann durch diese Ausgestaltung Ermüdungsbrüchen entgegengewirkt werden.

Da die Form der Leiterschleife grundsätzlich beliebig gewählt werden kann, bietet es sich an, daß bei einer biologischen Herzklappe die Leiterschleife die Form einer natürlichen Herzklappe hat und wenigstens zwei Schlaufen der Leiterschleife vorgesehen sind, die mit ihren Flächen eine Zylinderform bilden, die natürliche Klappenform umschließen und in den Bereichen zwischen den Schlaufen die Abgänge der Herzkranzgefäße freihalten.

Um eine kompakte Prothese zu erhalten, bietet es sich an, daß die zum Resonanzschwingkreis gehörende weitere Kapazität auf und/oder in der Klappenprothese befestigt bzw. angebracht ist und/oder in das Design der Klappenprothese integriert ist.

Dabei ist es im Zusammenhang mit der Verankerung so, daß die Induktivität und/oder die Kapazität des Resonanzschwingkreises zumindest teilweise zur Integration in das die Klappenprothese umhüllende Gewebe zur Annaht, Verankerung und/oder Abdichtung vorgesehen ist.

Zur Einstellung einer externen parasitären Kapazität ist die Leiterschleife mit einem Nichtleiter als Isolationsschicht, insbesondere mit Kunststoff und/oder Keramik, beschichtet. Damit läßt sich dann zum einen über die Auswahl und Technik der Beschichtung und zum anderen über die Dicke der Beschichtung, die mindestens 1 Nanometer aufweist, vorzugsweise aber zwischen 1 Nanometer bis 200 Mikrometer liegt, die gewünschte parasitäre Kapazität einstellen. Darüber hinaus wird durch die Isolierungsschicht und den Abstand zwischen den Schlaufen der wenigstens einen Leiterbahn eine interne Kapazität ausgebildet, wobei sich deren Größe wiederum durch die Art der Isolierungsschicht und den Abstand der einzelnen Schlaufen einstellen läßt.

Die äußere Beschichtung bzw. Isolationsschicht besteht dabei vorzugsweise aus Teflon, Polyester, Polyurethan, Parylene oder anderen geeigneten Polymeren. Neben der Isolierfunktion und der Ausbildung der parasitären und internen Kapazität kann die Isolationsschicht die zusätzliche Aufgabe haben, eine weitere zum Resonanzschwingkreis gehörende Spule und/oder wenigstens eine Kapazität auf und/oder in dem Gerüst der Prothese zu befestigen bzw. anzubringen.

Die Klappenprothese bzw. das Klappengerüst kann mit einem entsprechenden Material beschichtet werden.

Letztlich ist der Resonanzschwingkreis der erfindungsgemäßen Herzklappenprothese derart ausgebildet, daß der Schwingkreis eine Resonanzfrequenz, insbesondere im hochfrequenten Bereich, nämlich im Bereich zwischen 8 bis 400 MHz, besitzt, die der Frequenz eines äußeren Magnetfeldes, insbesondere eines MR-Tomographen entspricht.

Grundsätzlich kann vorgesehen sein, daß die Leiterschleife aus einem Draht oder einem Rohr oder aus Blech geschnitten aus einem Material mit einer geringen magnetischen Suszeptibilität, wie beispielsweise Nitinol oder einer guten elektrischen Leitfähigkeit, wie ein Edelmetall oder eine Edelmetallegierung oder aus einem Material mit einer geringen magnetischen Suszeptibilität und einer guten elektrischen Leitfähigkeit, wie beispielsweise Tantal, hergestellt ist. Alternativ ist es aber auch möglich, daß die Leiterschleife einen nicht leitenden Leiterträger aufweist, der mittels eines leitfähigen Materials, insbesondere Gold, Platin, Tantal und/oder leitfähigen Legierungen, beschichtet ist. Im übrigen ist es aber auch möglich, daß der Leiterträger elektrisch leitfähig ist und zur Verbesserung seiner elektrischen Leitfähigkeit mit einem entsprechenden Material beschichtet ist. Günstig ist es, die Leiterschleife durch eine Beschichtung der Klappenprothese mit einem leitenden Material zu realisieren. Bevorzugt erfolgt das Aufbringen der Beschichtung über eine Bedampfung oder ein Besputtern (PVD oder CVD).

Eine geringe magnetische Suszeptibilität liegt bei Materialien vor, die eine Dielektrizitätskonstante von sehr viel größer als 1 haben. So liegt beispielsweise die Dielektrizittskonstante der magnetischen Suszeptibilität von Nitinol bei 3· 10⁶. Demgegenüber weisen Materialien mit einer guten elektrischen Leitfähigkeit in [S/m] Werte auf, die sehr viel größer als 1 sind. So beträgt die elektrische Leitfähigkeit von Titan 2,34 · 106 S/m, während die von Kupfer zwischen 35 bis 58 · 10⁶ S/m liegt.

Im Zusammenhang mit dem Aufbringen der Beschichtung bietet es sich insbesondere bei Polymeren, Keramik, Nickeltitan und Titan als Material des Leiterträgers an, eine Basisschicht auf dieses Material aufzubringen, um die Haftung der Beschichtung zu verbessern. Bei der Basisschicht handelt es sich dann letztlich um einen Haftvermittler.

Zusätzlich zu der zuvor beschriebenen parasitären und/oder inneren Kapazität bietet es sich im übrigen an, daß eine zusätzliche Kapazität mittels eines Kondensators realisiert wird. Hierdurch ist es dann nicht erforderlich, besondere Formen oder besondere Beschichtungen der Leiterschleife zu realisieren, um vorgegebene innere und parasitäre Kapazitäten zu erzielen.

Zur Erleichterung der Implantation und um sowohl das Auffalten als auch die Funktion der erfindungsgemäßen Klappenprothese verfolgen zu können, ist bei einer faltbaren bzw. entfaltbaren Klappenprothese vorgesehen, daß diese derart ausgebildet ist, daß beim Entfalten die Resonanzfrequenz durch die Änderung der Spulengeometrie des Schwingkreises verändert wird und auf die Resonanzfrequenz des MR-Tomographen einstellbar ist.

Darüber hinaus betrifft die vorliegende Erfindung eine perkutan implantierbare und expandierbare, biologische oder künstliche Klappenprothese zur Verwendung im menschlichen und/oder tierischen Körper zum Ersatz einer Organklappe oder Gefäßklappe, mit mindestens einem mit einer Klappe versehenen Klappenmechanismus, und mit einem Stent mit einer Hülse aus biokompatiblem Material, wobei der Stent in der Funktionshöhe der Klappe von wenigstens einer Leiterschleife, die die Induktivität eines elektrischen Resonanzschwingkreises ausbildet, umschlossen ist.

Im Gegensatz zu der zuvor beschriebenen Ausführungsform wird nicht die Klappenprothese selbst bzw. das Klappengerüst oder aber die Klappe von der Leiterschleife gebildet. Bei dieser Ausführungsform ist es so, daß der Resonanzschwingkreis in Form der wenigstens einen Leiterschleife um den Stent herum in Funktionshöhe der Klappe angebracht ist.

Da das Traggerüst des Stents die MR-Funktion beeinträchtigen kann, wird das Traggerüst des Stents von der Leiterschleife gebildet. Eine negative Beeinflussung der Resonanzfrequenz durch ein separates Traggerüst kann in diesem Fall nicht auftreten. Außerdem ist es möglich, daß die Leiterschleife zur Befestigung des Klappenmechanismus im Stent dient und dazu zumindest teilweise aus dem Stent herausgeführt ist. Schließlich kann über die Leiterschleifer bzw. entsprechende Verankerungsmittel der Leiterschleife eine Verankerung des Stents im Klappenlager erzielt werden. Erkennbar hat auch hierbei die Leiterschleife dann eine Mehrfachfunktion,

Im Zusammenhang mit der zuvor beschriebenen Ausführungsform mit Stent versteht es sich, daß die diesbezügliche Klappenprothese alle vorgenannten Merkmale für sich oder aber in Kombination aufweisen kann, die zuvor bei der eingangs genannten Klappenprothese beschrieben worden sind.

Nachfolgend werden Ausführungsbeispiele der Erfindung anhand der Zeichnung erläutert. Dabei zeigt
- Fig. 1: eine perspektivische, schematische Ansicht einer biologischen Klappenprothese,
- Fig. 2: perspektivische, schematische Ansicht von weiteren Ausführungsformen biologischer Klappenprothesen,
- Fig. 3: eine perspektivische, schematische Ansicht einer künstlichen Klappenprothese mit einer als Deckel ausgebildeten Klappe auf Basis einer Solenoidspule,
- Fig. 4: eine der Fig. 3 entsprechende Ansicht einer Klappenprothese mit einer als Kugel ausgebildeten Klappe,
- Fig. 5: eine der Fig. 3 entsprechende Ansicht einer Klappenprothese mit einer als Doppelklappe ausgebildeten Klappe,
- Fig. 6: eine perspektivische, schematische Ansicht einer künstlichen Klappenprothese mit einer als Deckel ausgebildeten Klappe auf Basis einer Sattelspule,
- Fig. 7: eine der Fig. 6 entsprechende Ansicht einer künstlichen Klappenprothese mit einer als Kugel ausgebildeten Klappe,
- Fig. 8: eine der Fig. 6 entsprechende Ansicht einer künstlichen Klappenprothese mit einer als Doppelklappe ausgebildeten Klappe,
- Fig. 9: eine perspektivische, schematische Ansicht einer künstlichen Klappenprothese mit einer als Deckel ausgebildeten Klappe auf Basis einer Solenoidspule mit einer mäanderförmig geformten Leiterschleife,
- Fig. 10: eine der Fig. 9 entsprechende Ansicht einer künstlichen Klappenprothese mit einer als Kugel ausgebildeten Klappe,
- Fig. 11: eine der Fig. 9 entsprechende Ansicht einer künstlichen Klappenprothese mit einer als Doppelklappe ausgebildeten Klappe,
- Fig. 12: eine perspektivische, schematische Ansicht einer biologischen Klappenprothese in einem Stent mit einer Sattelspule,
- Fig. 13: eine perspektivische, schematische Ansicht einer künstlichen Klappenprothese in einem Stent mit einer Sattelspule,
- Fig. 14: eine perspektivische, schematische Ansicht einer künstlichen Klappenprothese in einem Stent mit einer als Kugel ausgebildeten Klappe,
- Fig. 15: eine der Fig. 13 entsprechende Ansicht einer künstlichen Klappenprothese mit einer als Doppelklappe ausgebildeten Klappe,
- Fig. 16: eine perspektivische, schematische Ansicht einer biologischen Klappenprothese mit einem Stent und einer äußeren Solenoidspule aus einem zu einem Mäander geformten elektrisch leitenden Material,
- Fig. 17: eine perspektivische, schematische Ansicht einer biologischen Herzklappe in einem Stent mit einer äußeren Zylinderspule,
- Fig. 18: eine perspektivische, schematische Ansicht einer künstlichen Klappenprothese mit einem Stent und einer äußeren Zylinderspule sowie einer als Deckel ausgebildeten Klappe,
- Fig. 19: eine der Figur 18 entsprechende Ansicht einer künstlichen Klappenprothese mit einer als Kugel ausgebildeten Klappe,
- Fig. 20: eine der Fig. 18 entsprechende Ansicht einer künstlichen Klappenprothese mit einer als Doppelklappe ausgebildeten Klappe,
- Fig. 21: eine perspektivische, schematische Ansicht einer künstlichen Klappenprothese in einem Stent mit einer äußeren Solenoidspule aus einem zu Mäander geformten elektrisch leitenden Material und einer als Deckel ausgebildeten Klappe,
- Fig. 22: eine der Fig. 21 entsprechende Ansicht einer künstlichen Klappenprothese mit einer als Kugel ausgebildeten Klappe,
- Fig. 23: eine der Fig. 21 entsprechende Ansicht einer künstlichen Klappenprothese mit einer als Doppelklappe ausgebildeten Klappe,
- Fig. 24: eine schematische Ansicht einer in einem Katheder angeordneten Klappenprothese,
- Fig. 25: eine schematische Ansicht einer durch einen Ballon expandierbaren Klappenprothese, und
- Fig. 26: verschiedene Darstellungen von Stents bzw. Klappenprothesen mit zellengleicher oder unterschiedlicher Größe.

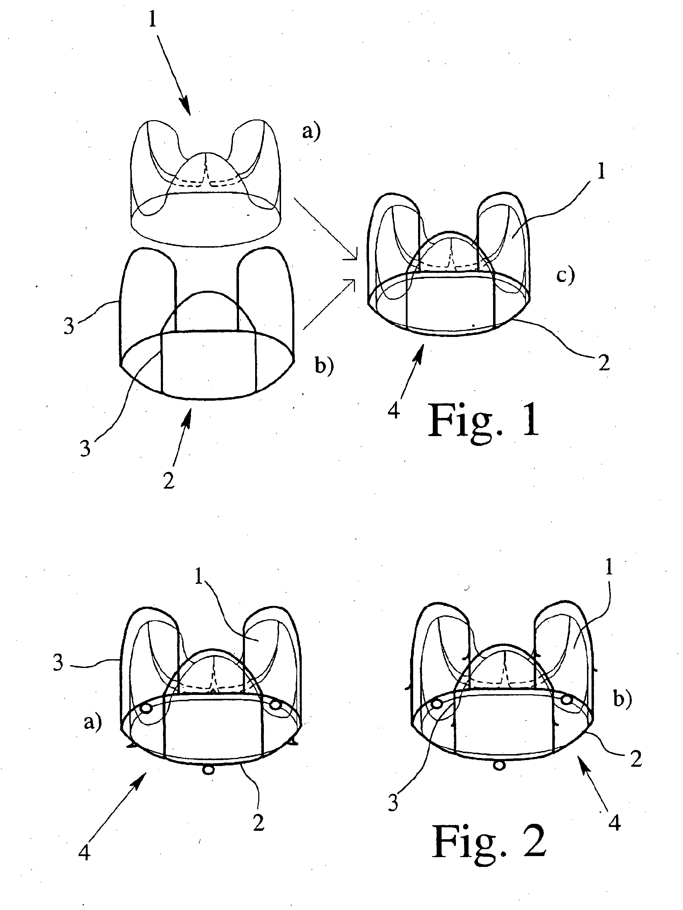

In der Darstellung a der Fig. 1 ist eine biologische Herzklappe 1 dargestellt. Die Art und Ausbildung der biologischen Herzklappe 1 an sich ist bekannt. In der Darstellung b ist eine Leiterschleife 2 gezeigt, die die Induktivität eines elektrischen Resonanzschwingkreises und damit den Resonanzschwingkreis als solchen ausbildet. Ein Vergleich der Darstellungen a und b der Fig. 1 ergibt, daß die Leiterschleife 2 als geschlossene Leiterbahn drei Schlaufen 3 ausbildet, die zusammen zylinderförmig angeordnet sind. Die Schlaufen 3 der Leiterschleife 2 sind der Form der biologischen Herzklappe 1 angepaßt.

Die Darstellung c aus Fig. 1 zeigt eine nicht erfindungsgemäße biologische Klappenprothese 4, wobei die Leiterschleife 2 bzw. der Schwingkreis in die biologische Herzklappe integriert sind bzw. die biologische Herzklappe 1 in die Leiterschleife 1 eingesetzt ist. Im einzelnen nicht dargestellt ist, daß die Leiterschleife 2 wenigstens eine Kapazität aufweist.

Die in Fig. 2 dargestellten Klappenprothesen 4 unterscheiden sich von der in Fig. 1 c) dargestellten Klappenprothese 4 dadurch, daß Verankerungsmittel in Form von Ösen und Haken vorgesehen sind. Bei der Ausführungsform gemäß Fig. 2 a) befinden sich Haken und Ösen lediglich am umlaufenden Ringbereich der Leiterschleife 2, während bei der Ausführungsform gemäß Fig. 2 b)

Verankerungsmittel vorliegend in Form von Haken ebenfalls an den Schlaufen vorgesehen sind.

Nicht dargestellt ist im übrigen, daß die Herzklappenprothese statt dreier Schlaufen auch nur zwei Schlaufen haben kann, um damit an eine Doppelsegelklappe angepaßt zu sein.

Weiterhin versteht es sich, daß die in Fig. 2 dargestellten Verankerungsmittel beispielsweise in Form von Haken und/oder Ösen bei allen nachfolgend dargestellten Leiterschleifen realisiert sein können, auch wenn dies im einzelnen nicht dargestellt ist.

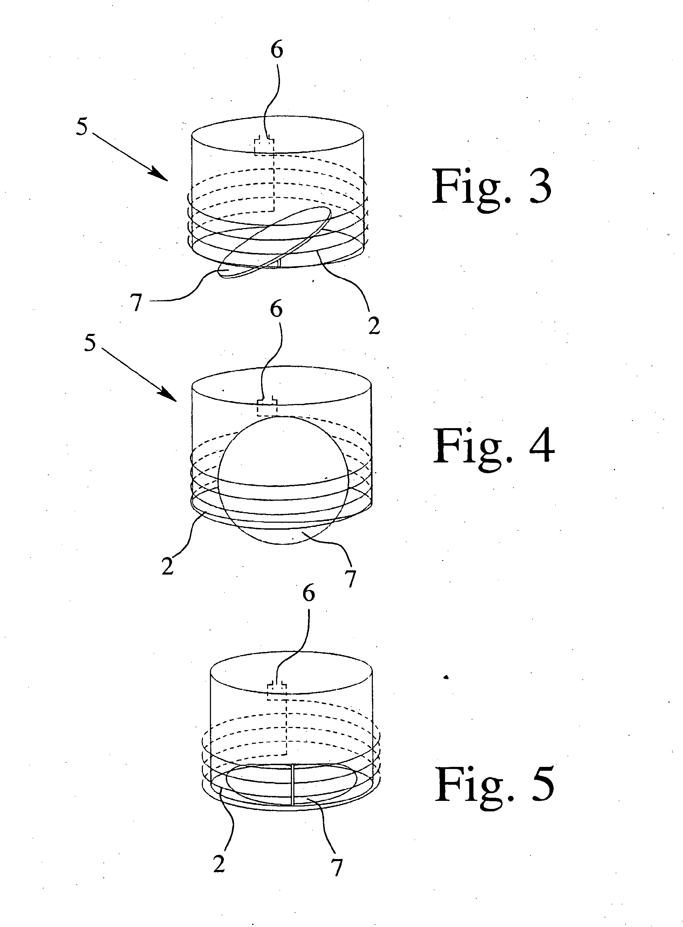

In Fig. 3 ist eine künstliche Klappenprothese 5 mit einer Leiterschleife 2 auf Basis einer Solenoidspule dargestellt. Der diesbezügliche Schwingkreis weist eine Kapazität 6 auf. Die künstliche Klappenprothese 5 weist eine als Deckel ausgebildete Klappe 7 auf. Im dargestellten Ausführungsbeispiel bildet die Leiterschleife 2 das tragende Klappengerüst der Klappenprothese 5. In diesem Zusammenhang darf darauf hingewiesen werden, daß es sich bei den einzelnen Darstellungen lediglich um schematische schaltbildartige Abbildungen der betreffenden Klappenprothesen 4, 5 handelt.

Die in Fig. 4 dargestellte künstliche Klappenprothese 5 entspricht der in Fig. 3 dargestellten Klappenprothese 5, wobei jedoch als Klappe 7 eine Kugel vorgesehen ist.

Die in Fig. 5 dargestellte künstliche Klappenprothese 5 entspricht ebenfalls der in Fig. 3 dargestellten Klappenprothese 5, wobei als Klappe 7 jedoch eine zweiflüglige Klappe nach dem Zweiklappenprinzip, die nachfolgend als Doppelklappe bezeichnet wird, vorgesehen ist.

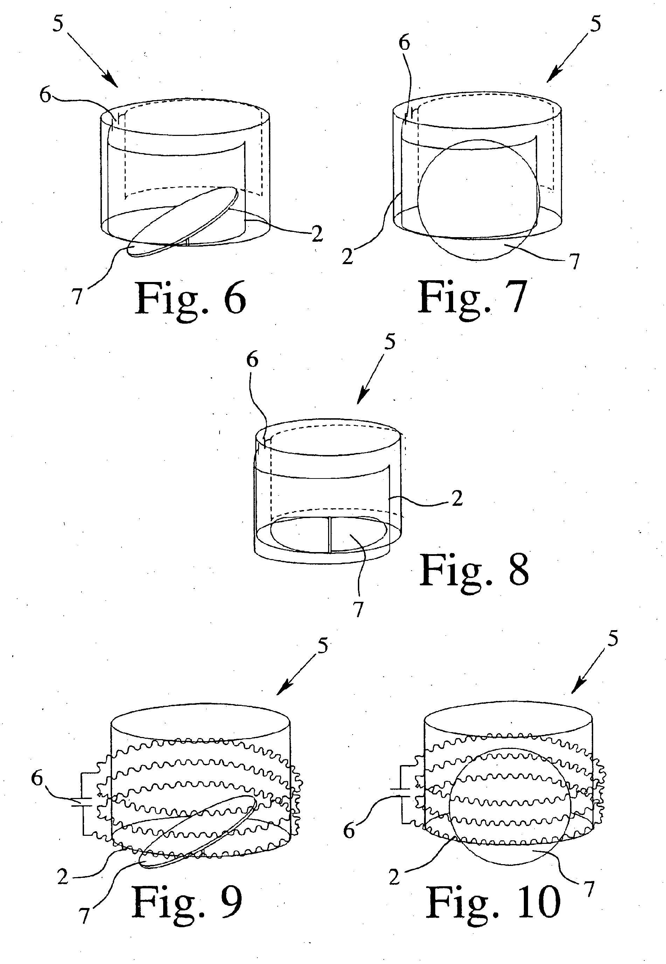

Die in Fig. 6 dargestellte Klappenprothese 5 entspricht im wesentlichen der in Fig. 3 dargestellten Klappenprothese 5, wobei jedoch statt einer Solenoidspule eine Sattelspule vorgesehen ist.

Die in Fig. 7 dargestellte Klappenprothese 5 entspricht der in Fig. 6 dargestellten, wobei jedoch als Klappe 7 eine Kugel vorgesehen ist.

Die in Fig. 8 dargestellte Klappenprothese 5 entspricht der in Fig. 6 dargestellten, wobei jedoch als Klappe 7 eine Doppelklappe vorgesehen ist.

Bei der in Fig. 9 dargestellten künstlichen Klappenprothese 5 ist ein integrierter Resonanzschwingkreis auf Basis einer Solenoidspule dargestellt, wie dies auch aus Fig. 3 hervorgeht. Im Unterschied zu der Ausführungsform gemäß Fig. 3 ist die Leiterschleife 2 mäanderförmig ausgebildet. Die Mäanderform des Leiterdrahtes ermöglicht es in besonders einfacher Weise, die Klappenprothese 5 zusammenzufalten.

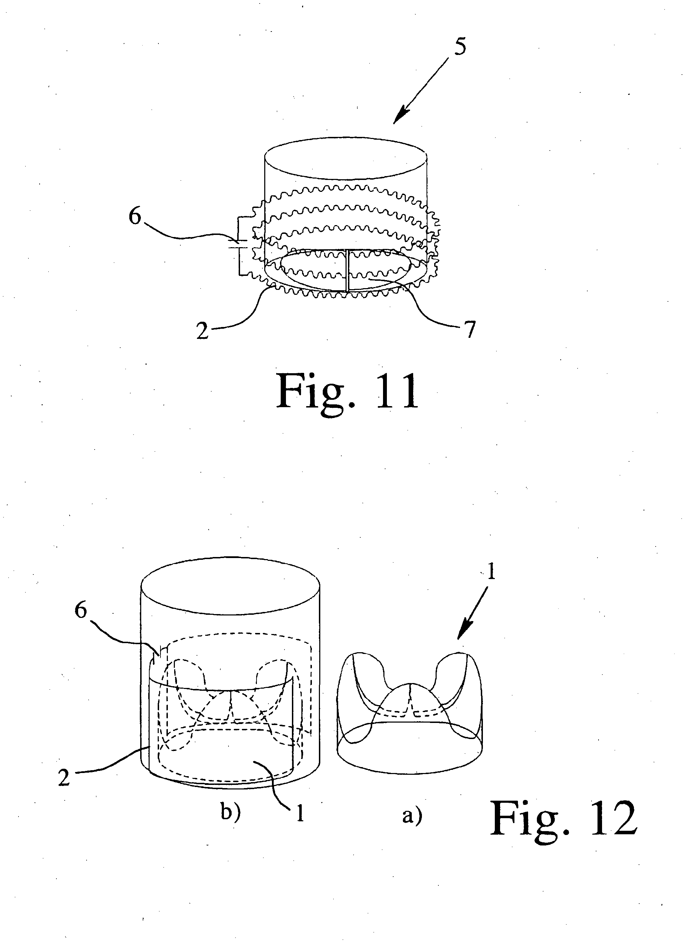

Die Ausführungsform gemäß Fig. 10 entspricht der Ausführungsform gemäß Fig. 6, wobei als Klappe 7 jedoch eine Kugel vorgesehen ist. Bei der in Fig. 11 gezeigten Ausführungsform ist im Unterschied zu der in Fig. 10 dargestellten Ausführungsform statt der Kugel eine Doppelklappe vorgesehen.

In Fig. 12 ist in der Darstellung a eine biologische Herzklappe 1 gezeigt. Die Herzklappe 1 ist, wie sich aus der Darstellung b ergibt, in einen Stent 8 integriert. Im übrigen ist als Träger der Herzklappe 1 und damit als Gerüst für die Herzklappe 1 eine als Sattelspule ausgebildete Leiterschleife 2 vorgesehen, die eine Kapazität 6 aufweist. Die Höhe der Leiterschleife 6 entspricht der Höhe der Herzklappe 1, so daß alle relevanten Bereiche über die Leiterschleife 2 erfaßt werden können. Im übrigen versteht es sich, daß zusätzlich zu der Sattelspule eine weitere Leiterschleife 2 vorgesehen sein kann, die in Fig. 1b dargestellt ist. Dabei können die einzelnen Spulen aus unterschiedlichen Leiterschleifen 2 gebildet sein oder aus einer einzigen Leiterschleife 2.

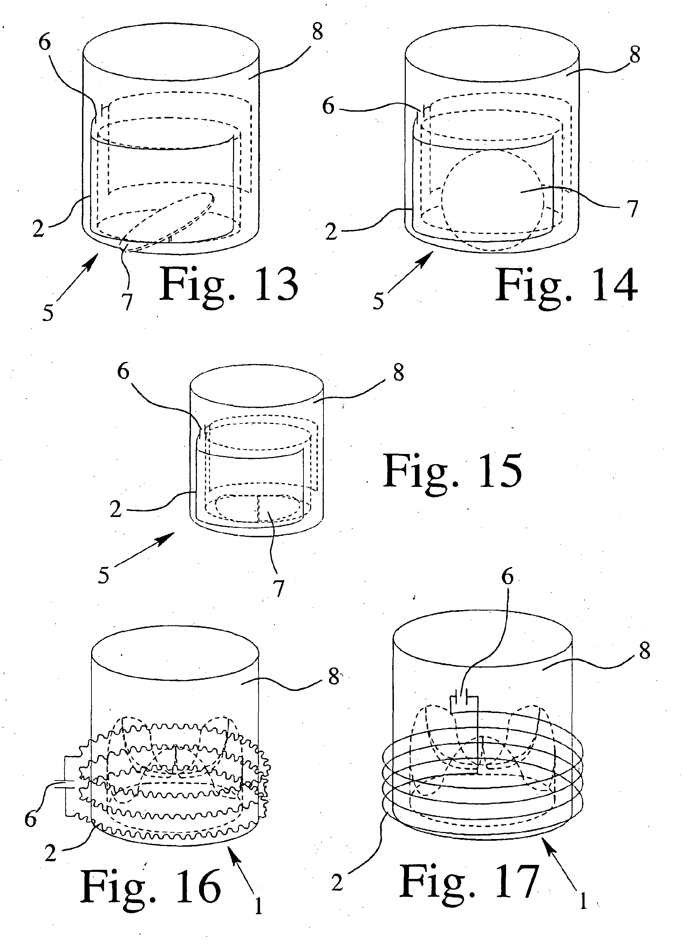

Die Ausführungsformen der Fig. 13 bis 15 entsprechen im wesentlichen der Ausführungsform der Fig. 12, wobei es sich bei den in den Fig. 13 bis 15 dargestellten Ausführungsformen um künstliche Klappenprothesen 5 handelt, die in einem Stent 8 angeordnet sind. Die jeweilige Leiterschleife 2 ist jeweils als Sattelspule ausgebildet und innerhalb des Stents 8 angeordnet. Die Leiterschleife 2 bildet jeweils den Träger der Klappenprothese 5, wobei als Klappe 7 bei der Ausführungsform gemäß Fig. 13 ein Deckel, bei der Ausführungsform gemäß Fig. 14 eine Kugel und bei der Ausführungsform gemäß Fig. 15 eine Doppelklappe vorgesehen ist.

In den Fig. 16 bis 23 sind jeweils Ausführungsformen dargestellt, bei denen die jeweilige Klappenprothese 4, 5 einen Stent 8 aufweist, in den eine biologische Herzklappe 1 oder aber eine künstliche Herzklappe 9 als Klappenmechanismus integriert ist. Der Stent 8 weist jeweils eine Hülse aus einem biokompatiblen Material auf. Im übrigen ist der Stent 8 in Funktionshöhe der jeweiligen Klappe von mindestens einer Leiterschleife 2, die die Induktivität eines elektrischen Resonanzschwingkreises ausbildet, umschlossen. Dabei ist es bei allen, auch den zuvor dargestellten Ausführungsformen so, daß die Höhe der jeweiligen Spule bzw. die Höhenerstreckung der Leiterschleife derart, daß sich ein solches relevantes Spulenfeld ergibt, daß die jeweilige Klappe auch während der Funktion im wesentlichen vollständig eingeschlossen ist. Das Verhältnis des Durchmessers der Prothesenöffnung zur Höhe der jeweiligen Spule liegt zwischen 4 : 1 bis 0,5 : 1.

Auch bei den Fig. 16 bis 23 handelt es sich lediglich um schematische Darstellungen. Bei allen dargestellten Ausführungsformen sind folgende Optionen möglich:
1. Die Leiterschleife 2 bildet das Traggerüst des Stents 8 aus. Hierbei übernimmt dann die Leiterschleife 2 nicht nur die Funktion des Resonanzschwingkreises, sondern auch die Gerüstfunktion des Stents.
2. Die Leiterschleife 2 ist nicht nur um den Stent 8 im relevanten Bereich gewickelt, sondern dient auch zur Befestigung der biologischen Herzklappe 1 bzw. der künstlichen Herzklappe 9. Letztlich ist die Herzklappe 1, 9 als Klappenmechanismus mit der Leiterschleife 2 oder Teilen der Leiterschleife 2 vernäht. In diesem Falle übernimmt die Leiterschleife 2 die zusätzliche Funktion der Befestigung der Herzklappe 1, 9 innerhalb der Hülse des Stents 8. Dabei können dann nach außen geführte Bereiche der Leiterschleife 2 Haken, Ösen oder andere Verankerungsmittel aufweisen, um die Befestigung des Stents 8 an der Implantationsstelle zu erleichtern.
3. Darüber hinaus sind Kombinationen aus zwei der vorgenannten Möglichkeiten gegeben. So kann die Leiterschleife 2 zusätzlich zu der Alternative 2 auch, zumindest im relevanten Bereich der Herzklappe 1, 9, die Traggerüstfunktion des Stents 8 erfüllen.

Die Fig. 16 und 17 zeigen jeweils eine biologische Herzklappe 1, die in den Stent 8 eingesetzt ist. Im relevanten Bereich, d. h. im Bereich der Herzklappe 1, befindet sich bei der Ausführungsform gemäß Fig. 16 eine Solenoidspule mit einer mäanderförmig geformten Leiterschleife 2, während bei der Ausführungsform gemäß Fig. 17 die Spule als Zylinderspule ausgeführt ist.

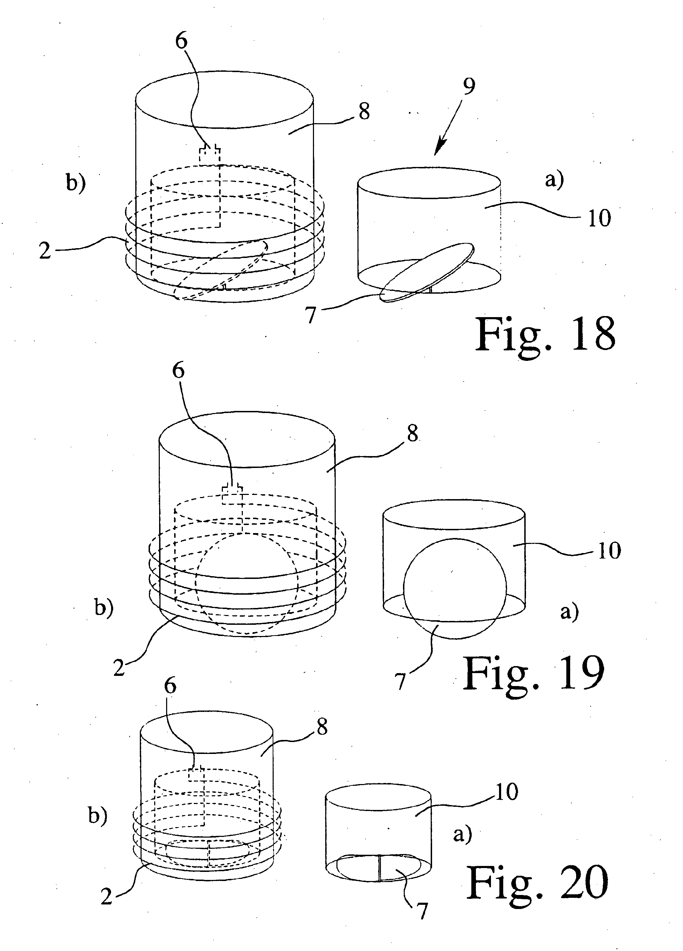

Bei der in Fig. 18 dargestellten Ausführungsform ist eine künstliche Herzklappe 9 vorgesehen, die einen Träger 10 aufweist, der zur Lagerung der Klappe 7 dient, was in Fig. 18a dargestellt ist. Bei der Darstellung gemäß Fig. 18b ist die künstliche Herzklappe 9 in den Stent 8 eingesetzt, wobei die Leiterschleife 2 eine Zylinderspule mit Kapazität 6 bildet.

Die in Fig. 19 dargestellte Ausführungsform entspricht im wesentlichen der in Fig. 18 dargestellten Ausführungsform, wobei als Klappe 7 eine Kugel vorgesehen ist. Bei der in Fig. 20 dargestellten Ausführungsform ist hingegen als Klappe 7 eine Doppelklappe vorgesehen.

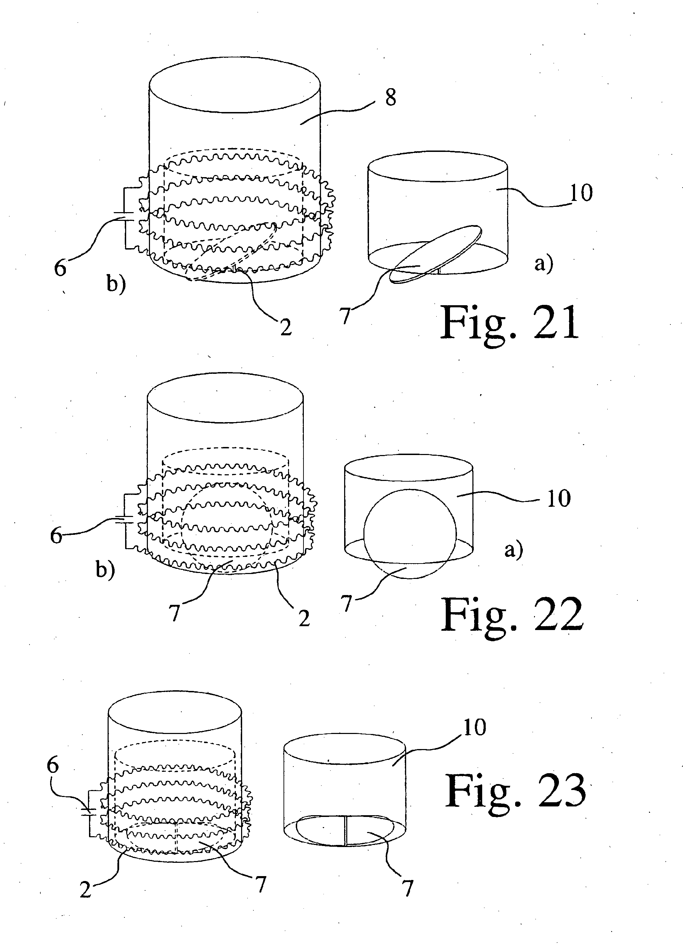

Die in Fig. 21 dargestellte Ausführungsform entspricht der in Fig. 18 dargestellten Ausführungsform, wobei die Leiterschleife 2 als Solenoidspule und die Leiterbahn 2 als solche mäanderförmig ausgebildet sind.

Während bei der Ausführungsform gemäß Fig. 21 ein Deckel als Klappe 7 vorgesehen ist, ist bei der Ausführungsform gemäß Fig. 22 eine Kugel als Klappe vorgesehen. Demgegenüber ist bei der Ausführungsform gemäß Fig. 23 als Klappe eine Doppelklappe vorgesehen. Ansonsten bestehen zwischen den Ausführungsformen gemäß den Fig. 21, 22 und 23 keine Unterschiede.

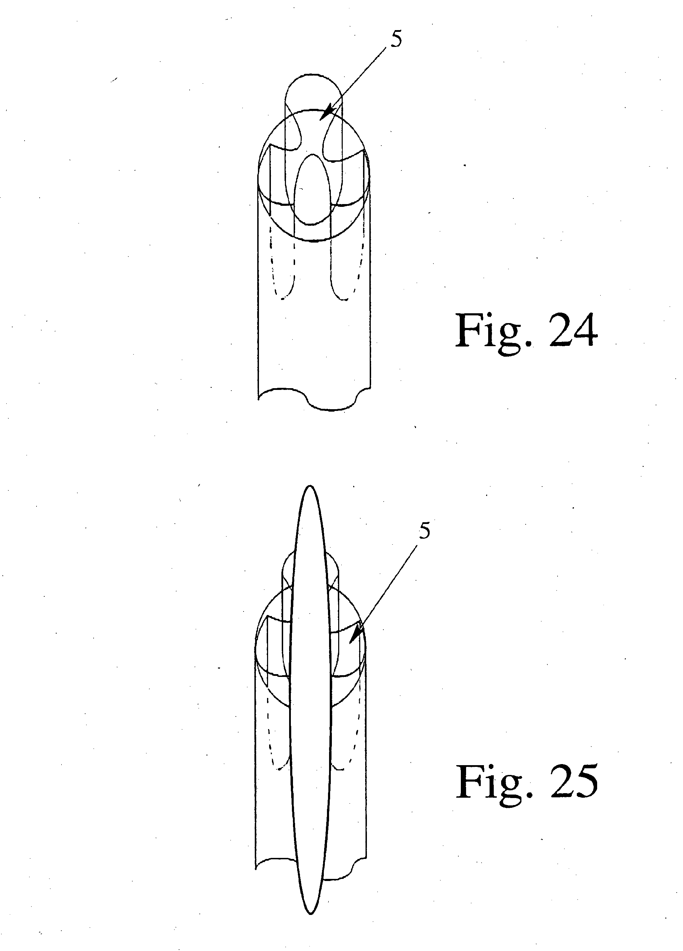

In den Fig. 24 und 25 ist schematisch das Einsetzen einer erfindungsgemäßen Klappenprothese 5 dargestellt. Bei der in Fig. 24 dargestellten Klappenprothese 5 handelt es sich um eine selbst expandierende Prothese, die nach Anordnung an der Implantationsstelle und Zurückziehen des Katheters, der die Prothese im zusammengefalteten Zustand hält, selbst expandiert. Demgegenüber wird die Prothese 5 der in Fig. 25 dargestellten Ausführungsform über einen Ballon, der innerhalb der Prothese angeordnet ist, expandiert. Hierzu wird die Prothese an die Implantationsstelle mit einem Zuführkatheter gebracht, der anschließend zurückgezogen wird. Nach Zurückziehen des Katheders faltet sich die Prothese zunächst nicht auf. Die Auffaltung wird durch Expansion des Ballons erzeugt. Hierzu wird dem Ballon ein entsprechendes Medium zugeführt. Nach der Expansion wird der Ballon aus der Prothese herausgezogen.

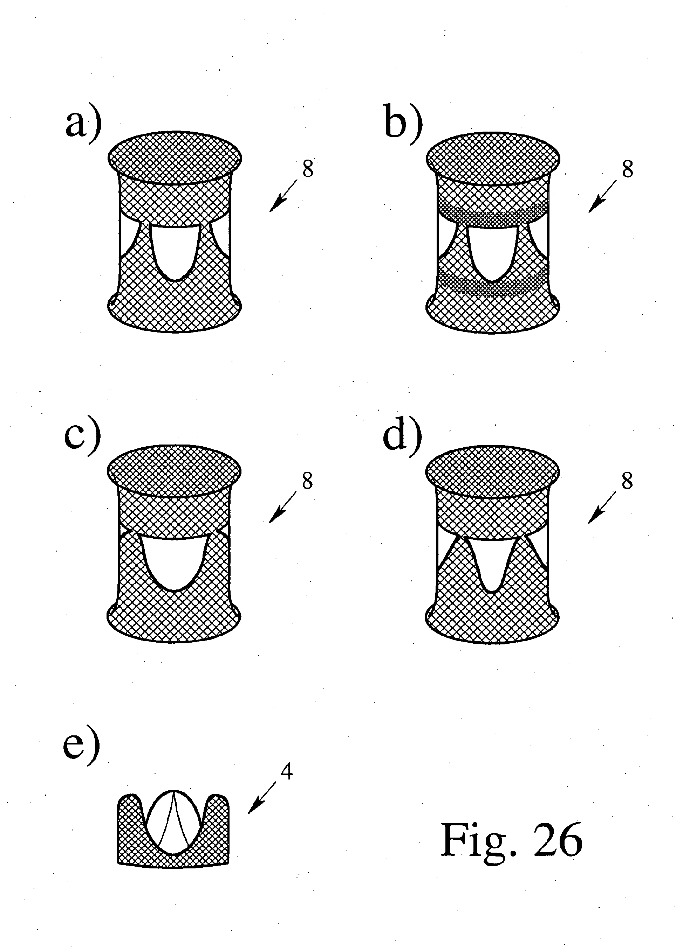

Fig. 26 zeigt verschiedene Ausführungsformen erfindungsgemäßer Klappenprothesen. Die Ausführungsformen a), b), c) und d) zeigen dabei jeweils einen Stent 8, wobei die äußere umlaufende Mantelfläche des Stents 8 gitter- oder netzförmig aufgebaut ist und dabei jeweils eine Vielzahl von Zellen mit gleicher und mit unterschiedlicher Größe aufweist. Grundsätzlich können die Zellen, die auch als Fenster oder Öffnungen bezeichnet werden können, jegliche Form haben.

Bei der Ausführungsform gemäß Fig. 26 a sind zwei Typen von Zellen vorgesehen, nämlich überwiegend kleine Zellen und im mittleren Bereich größere, etwa dreieckförmige Zellen.

Die Ausführungsform gemäß Fig. 26b unterscheidet sich von der gemäß Fig.26 a dadurch, daß drei Arten von Zellen mit unterschiedlichen Größen vorgesehen sind. Unterhalb und oberhalb der großen mittleren Zellen befindet sich dabei jeweils ein Streifen mit sehr kleinen Zellen.

Bei der Ausführungsform gemäß Fig. 26c sind die mittleren Zellen relativ groß. Es sind lediglich drei große Zellen vorgesehen.

Die Ausführungsform gemäß Fig. 26 d entspricht im wesentlichen der gemäß Fig. 26 c, wobei jedoch mehr als drei große mittlere Zellen vorgesehen sind.

Fig. 26e zeigt eine Klappenprothese (4), die im wesentlichen der Klappenprothese gemäß Fig. 1 c entspricht, wobei jedoch außenseitig eine Vielzahl von kleinen Zellen vorgesehen sind.

## Patentansprüche

1. Biologische oder künstliche Klappenprothese (4, 5) zur Verwendung im menschlichen oder tierischen Körper zum Ersatz einer Organklappe oder Gefäßklappe, insbesondere Herz- oder Venenklappenprothese, mit einem Stent (8) oder stentlos, mit einem tragenden Klappengerüst, mit wenigstens einer Klappe (7) und mit mindestens einer Leiterschleife (2), die die Induktivität eines elektrischen Resonanzschwingkreises ausbildet,
**dadurch gekennzeichnet,**
**daß** die wenigstens eine Leiterschleife (2) das Klappengerüst selbst und/oder die Klappe (7) selbst ausbildet und eine Tragfunktion bei der Klappenprothese übernimmt und daß die Leiterschleife (2) mit einem Nichtleiter als Isolationsschicht beschichtet ist.

2. Klappenprothese nach Anspruch 1, **dadurch gekennzeichnet, daß** der Stent (8) und/oder das Klappengerüst außenseitig eine Mehrzahl von Zellen mit gleicher und/oder unterschiedlicher Größe aufweist.

3. Klappenprothese nach Anspruch 1 oder 2, **dadurch gekennzeichnet, daß** die Klappenprothese (4, 5) falt- und expandierbar und damit perkutan implantierbar ausgebildet ist.

4. Klappenprothese nach einem der vorhergehenden Ansprüche, **dadurch gekennzeichnet, daß** an der Leiterschleife (2) und/oder dem Klappengerüst Verankerungsmittel, vorzugsweise in Form von Ösen, Haken od. dgl. vorgesehen sind und daß, vorzugsweise, die Verankerungsmittel einstückig mit der Leiterschleife (2) ausgebildet sind.

5. Klappenprothese nach einem der vorangehenden Ansprüche, **dadurch gekennzeichnet, daß** die Verankerungsmittel in komprimiertem Zustand nicht über die Klappenprothese (4, 5) überstehen und erst nach der Entfaltung wirksam werden.

6. Klappenprothese nach einem der vorangehenden Ansprüche, **dadurch gekennzeichnet, daß** eine biologische stentlose Herzklappe (1), insbesondere vom Tier, wie vom Schwein (Herzklappe, Herzbeutel) bzw. vom Rind (Herzbeutel) oder von menschlichen Geweben und Zellen, von mindestens einer Leiterschleife (2) umschlossen ist.

7. Klappenprothese nach einem der vorangehenden Ansprüche, **dadurch gekennzeichnet, daß** bioresorbierbare Anteile, die die Klappenprothese (4, 5) für die Implantation stabilisieren, vorgesehen sind, daß die wenigstens eine Leiterschleife (2) zur Implantation in einem biologischen Anteil der Klappenprothese (4, 5) vorgesehen ist und dort verbleibt, nachdem die bioresorbierbaren Anteile im Körper abgebaut worden sind.

8. Klappenprothese nach einem der vorangehenden Ansprüche, **dadurch gekennzeichnet, daß** das Klappengerüst und die Klappe (7) in einem selbst entfaltbaren oder mechanisch expandierbaren Stent durch die Leiterschleife (2) zumindest teilweise derart fixiert sind, daß bei der Komprimierung und anschließenden Entfaltung sowohl das Klappengerüst und die Klappe (7) als auch die Leitersehleife (2) in die gewünschte Form, Position und Geometrie zurückkehren, wobei vorzugsweise die notwendige Abdichtung der Prothese im Lager erreicht wird.

9. Klappenprothese nach einem der vorhergehenden Ansprüche, **dadurch gekennzeichnet, daß** das Klappengerüst mehrere Resonanzschwingkreise mit jeweils einer Leiterschleife (2) ausgebildet oder daß mehrere Resonanzschwingkreise mit unterschiedlichen Resonanzfrequenzen und nur einer Leiterschleife (2) miteinander gekoppelt sind.

10. Klappenprothese nach einem der vorangehenden Ansprüche, **dadurch gekennzeichnet, daß** das Klappengerüst mehrere unterschiedliche Spulenformen, insbesondere als Zylinder-, Sattel- oder Birdcagespule ausgebildet, aufweist.

11. Klappenprothese nach einem der vorangehenden Ansprüche, **dadurch gekennzeichnet, daß** mindestens eine Spule des Resonanzschwingkreises faltbare Geometrien, gekreuzte Leiterschleifen oder eine Mäanderstruktur aufweist und daß, vorzugsweise, die Leiterbahn der Leiterschleife (2) mäanderförmig ausgebildet ist.

12. Klappenprothese nach einem der vorangehenden Ansprüche, **dadurch gekennzeichnet, daß** die Leiterschleife (2) die Form einer biologischen Herzklappe hat, und daß mindestens zwei Schlaufen der Leiterschleife (2) vorgesehen sind, die mit ihren Flächen eine Zylinderform bilden, die die natürliche Klappenform schlaufenförmig umschließen und in den Bereichen zwischen den Schlaufen die Abgänge der Herzkranzgefäße freihalten.

13. Klappenprothese nach einem der vorangehenden Ansprüche, **dadurch gekennzeichnet, daß** die zum Resonanzschwingkreis gehörende Kapazität (6) auf und/oder in der Klappenprothese (4, 5) befestigt/angebracht ist und/oder in das Design der Klappenprothese (4, 5) integriert ist.

14. Klappenprothese nach einem der vorangehenden Ansprüche, **dadurch gekennzeichnet, daß** die Induktivität und/oder die Kapazität (6) des Resonanzschwingkreises zumindest teilweise zur Integration in das die Klappenprothese (4, 5) umhüllende Gewebe zur Annaht, Verankerung und/oder Abdichtung vorgesehen sind.

15. Klappenprothese nach einem der vorangehenden Ansprüche, **dadurch gekennzeichnet, daß** die Leiterschleife (2) mit Kunststoff, wie Teflon, Polyester, Polyurethan, Parylene oder dgl. Polymere, und/oder mit Keramik beschichtet ist und daß, vorzugsweise, die Beschichtung eine Dicke von mindestens 1 Nanometer aufweist, insbesondere zwischen 1 Nanometer und 200 Mikrometer aufweist.

16. Klappenprothese nach einem der vorangehenden Ansprüche, **dadurch gekennzeichnet, daß** durch die Isolierungsschicht und den Abstand zwischen den Schlaufen der Leiterschleifen (2) eine interne Kapazität ausgebildet ist.

17. Klappenprothese nach einem der vorangehenden Ansprüche, **dadurch gekennzeichnet, daß** eine weitere Kapazität (6) des Schwingkreises mittels eines Kondensators realisiert wird.

18. Klappenprothese nach einem der vorangehenden Ansprüche, **dadurch gekennzeichnet, daß** eine weitere Spule und/oder die weitere Kapazität (6) auf und/oder in oder an dem Klappengerüst mit Hilfe der Isolationsschicht befestigt bzw. angebracht ist.

19. Klappenprothese nach einem der vorhergehenden Ansprüche, **dadurch gekennzeichnet, daß** die Leiterschleife (2) auf dem Klappengerüst mittels einer Schicht befestigt oder angebracht ist.

20. Klappenprothese nach einem der vorangehenden Ansprüche, **dadurch gekennzeichnet, daß** der Resonanzschwingkreis eine Resonanzfrequenz, insbesondere im Bereich von 8 bis 400 MHz, besitzt, die der Frequenz eines äußeren Magnetfeldes, insbesondere eines MR-Tomographen, entspricht.

21. Klappenprothese nach einem der vorangehenden Ansprüche, **dadurch gekennzeichnet, daß** die Leiterschleife (2) aus einem Draht oder einem Rohr oder aus Blech geschnitten, aus einem Material mit einer geringen magnetischen Suszeptibilität und/oder einer guten elektrischen Leitfähigkeit, hergestellt ist.

22. Klappenprothese nach einem der vorangehenden Ansprüche, **dadurch gekennzeichnet, daß** die Leiterschleife (2) einen elektrisch leitenden oder elektrisch nichtleitenden Leiterträger aufweist, der mittels eines leitfähigen Materials, insbesondere Gold, Platin, Tantal, Silber oder Kupfer und/oder leitfähiger Legierungen beschichtet ist.

23. Klappenprothese nach einem der vorangehenden Ansprüche, **dadurch gekennzeichnet, daß** die Leiterschleife (2) durch eine Beschichtung der Klappenprothese mit einem elektrisch leitenden Material realisiert wird und daß, insbesondere, das elektrisch leitende oder nichtleitende Material der Klappenprothese (4, 5) mit dem elektrisch leitenden Material bedampft oder besputtert ist.

24. Klappenprothese nach einem der vorangehenden Ansprüche, **dadurch gekennzeichnet, daß** insbesondere bei Polymeren, Keramik, Nickeltitan und Titan als Material des Leiterträgers auf die Leiterschleife (2) eine Basisschicht aufgebracht ist, um die Haftung einer Beschichtung zu verbessern.

25. Klappenprothese nach einem der vorangehenden Ansprüche, **dadurch gekennzeichnet, daß** die Klappenprothese (4, 5) faltbar bzw. entfaltbar und derart ausgebildet ist, daß beim Entfalten die Resonanzfrequenz durch die Änderung der Spulengeometrie des Schwingkreises verändert wird und auf die Resonanzfrequenz des MR-Tomographen einstellbar ist, so daß der Entfaltungsprozeß bei der Implantation über die Änderung der Signalcharakteristik im MR-Bild verfolgt werden kann.

26. Perkutan implantierbare und expandierbare, biologische oder künstliche Klappenprothese (4, 5), insbesondere nach einem der vorhergehenden Ansprüche, zur Verwendung im menschlichen und/oder tierischen Körper zum Ersatz einer Organklappe oder Gefäßklappe, mit mindestens einem mit einer Klappe (7) versehenen Klappenmechanismus, und mit einem eine Hülse aus biokompatiblem Material aufweisenden Stent (8), wobei der Stent (8) in der Funktionshöhe der Klappe von mindestens einer Leiterschleife (2), die die Induktivität eines elektrischen Resonanzschwingkreises ausbildet, umschlossen ist, **dadurch gekennzeichnet, dass** der Stent (8) ein Traggerüst aufweist und das Traggerüst von der Leiterschleife (2) gebildet wird und wobei die Leiterschleife (2) eine tragende Funktion bei der Klappenprothese übernimmt.

27. Klappenprothese nach Anspruch 26, **dadurch gekennzeichnet, daß** der Klappenmechanismus in dem Stent (8) mittels der Leiterschleife (2) an der Hülse des Stents (8) befestigt ist, und daß, vorzugsweise, aus der Hülse ragende Bereiche der Leiterschleife (2) als Verankerungsmittel, insbesondere als Haken und/oder Ösen, ausgebildet sind.

## Claims

1. Biological or artificial valve prosthesis (4, 5) for use in the human or animal body for the replacement of an organ valve or vessel valve, in particular a cardiac valve prosthesis or venous valve prosthesis, with or without a stent (8), with a supporting valve framework, with at least one valve (7) and with at least one conductor loop (2) which forms the inductance of an electrical resonant circuit,
**characterized in that**
the at least one conductor loop (2) forms the valve framework itself and/or the valve (7) itself and adopts a support function in the valve prosthesis, and **in that** the conductor loop (2) is coated with a non-conductor as insulating layer.

2. Valve prosthesis according to Claim 1, **characterized in that** the stent (8) and/or the valve framework has on the outside a plurality of cells of the same and/or different size.

3. Valve prosthesis according to Claim 1 or 2, **characterized in that** the valve prosthesis (4, 5) is collapsible and expandable and is thus designed to be percutaneously implantable.

4. Valve prosthesis according to one of the preceding claims, **characterized in that** anchoring means, preferably in the form of eyelets, hooks or the like, are provided on the conductor loop (2) and/or the valve framework, and **in that**, preferably, the anchoring means are formed in one piece with the conductor loop (2).

5. Valve prosthesis according to one of the preceding claims, **characterized in that** the anchoring means do not protrude beyond the valve prosthesis (4, 5) in the compressed state and only become active after deployment.

6. Valve prosthesis according to one of the preceding claims, **characterized in that** a biological stentless cardiac valve (1), in particular from an animal such as a pig (cardiac valve, pericardium) or a cow (pericardium) or from human tissues and cells, is enclosed by at least one conductor loop (2).

7. Valve prosthesis according to one of the preceding claims, **characterized in that** bioresorbable components which stabilize the valve prosthesis (4, 5) for the implantation are provided, and **in that** the at least one conductor loop (2) is provided, for the implantation, in a biological component of the valve prosthesis (4, 5) and remains there after the bioresorbable components have been broken down in the body.

8. Valve prosthesis according to one of the preceding claims, **characterized in that** the valve framework and the valve (7) are at least partially fixed by the conductor loop (2) in a self-deploying or mechanically expandable stent, in such a way that, during the compression and subsequent deployment, both the valve framework and the valve (7) and also the conductor loop (2) return to the desired shape, position and geometry, thereby preferably achieving the necessary sealing of the prosthesis in the anatomical bed.

9. Valve prosthesis according to one of the preceding claims, **characterized in that** the valve framework forms several resonant circuits, each with a conductor loop (2), or **in that** several resonant circuits with different resonant frequencies and only one conductor loop (2) are coupled to each other.

10. Valve prosthesis according to one of the preceding claims, **characterized in that** the valve framework has several different coil shapes, in particular designed as a cylindrical coil, saddle coil or birdcage coil.

11. Valve prosthesis according to one of the preceding claims, **characterized in that** at least one coil of the resonant circuit has collapsible geometries, crossed conductor loops or a meandering structure, and **in that**, preferably, the conductor track of the conductor loop (2) is designed with a meandering shape.

12. Valve prosthesis according to one of the preceding claims, **characterized in that** the conductor loop (2) has the shape of a biological cardiac valve, and **in that** at least two loops of the conductor loop (2) are provided which form a cylinder shape with their surfaces, which enclose the natural valve shape in the form of a loop, and which keep the origins of the coronary vessels free in the areas between the loops.

13. Valve prosthesis according to one of the preceding claims, **characterized in that** the capacitance (6) belonging to the resonant circuit is secured/mounted on and/or in the valve prosthesis (4, 5) and/or is integrated into the design of the valve prosthesis (4, 5).

14. Valve prosthesis according to one of the preceding claims, **characterized in that** the inductance and/or the capacitance (6) of the resonant circuit are provided at least in part for integration into the tissue enclosing the valve prosthesis (4, 5) for the purpose of sewing on, anchoring and/or sealing.

15. Valve prosthesis according to one of the preceding claims, **characterized in that** the conductor loop (2) is coated with plastic, such as Teflon, polyester, polyurethane, parylene or similar polymers, and/or is coated with ceramic, and **in that**, preferably, the coating has a thickness of at least 1 nanometre, in particular of between 1 nanometre and 200 micrometres.

16. Valve prosthesis according to one of the preceding claims, **characterized in that** an internal capacitance is formed by the insulating layer and the distance between the loops of the conductor loops (2).

17. Valve prosthesis according to one of the preceding claims, **characterized in that** a further capacitance (6) of the resonant circuit is implemented by means of a capacitor.

18. Valve prosthesis according to one of the preceding claims, **characterized in that** a further coil and/or the further capacitance (6) is secured or mounted on and/or in or on the valve framework with the aid of the insulating layer.

19. Valve prosthesis according to one of the preceding claims, **characterized in that** the conductor loop (2) is secured or mounted on the valve framework by means of a layer.

20. Valve prosthesis according to one of the preceding claims, **characterized in that** the resonant circuit has a resonant frequency, particularly in the range of 8 to 400 MHz, which corresponds to the frequency of an external magnetic field, in particular of an MR tomograph.

21. Valve prosthesis according to one of the preceding claims, **characterized in that** the conductor loop (2) is cut from a wire or a tube or from sheet metal, produced from a material with low magnetic susceptibility and/or with good electrical conductivity.

22. Valve prosthesis according to one of the preceding claims, **characterized in that** the conductor loop (2) has an electrically conductive or electrically non-conductive conductor support which is coated with a conductive material, in particular gold, platinum, tantalum, silver or copper and/or conductive alloys.

23. Valve prosthesis according to one of the preceding claims, **characterized in that** the conductor loop (2) is obtained through coating of the valve prosthesis with an electrically conductive material, and **in that**, in particular, the electrically conductive or non-conductive material of the valve prosthesis (4, 5) is coated with the electrically conductive material by vapour deposition or sputtering.

24. Valve prosthesis according to one of the preceding claims, **characterized in that**, particularly in the case of polymers, ceramic, nickel titanium and titanium as the material of the conductor support, a base layer is applied to the conductor loop (2) in order to improve the adhesion of a coating.

25. Valve prosthesis according to one of the preceding claims, **characterized in that** the valve prosthesis (4, 5) is collapsible and deployable and is designed in such a way that, upon deployment, the resonant frequency is changed by the change in the coil geometry of the resonant circuit and is adjustable to the resonant frequency of the MR tomograph, such that the deployment process during the implantation can be monitored via the change of the signal characteristic in the MR image.

26. Percutaneously implantable and expandable, biological or artificial valve prosthesis (4, 5), in particular according to one of the preceding claims, for use in the human and/or animal body for the replacement of an organ valve or vessel valve, with at least one valve mechanism provided with a valve (7), and with a stent (8) having a sleeve made of biocompatible material, wherein the stent (8) is enclosed at the functional height of the valve by at least one conductor loop (2) which forms the inductance of an electrical resonant circuit, **characterized in that** the stent (8) has a support framework, and the support framework is formed by the conductor loop (2), and wherein the conductor loop (2) adopts a supporting function in the valve prosthesis.

27. Valve prosthesis according to Claim 26, **characterized in that** the valve mechanism is secured in the stent (8) by means of the conductor loop (2) on the sleeve of the stent (8), and **in that**, preferably, areas of the conductor loop (2) protruding from the sleeve are designed as anchoring means, in particular as hooks and/or eyelets.

## Revendications

1. Prothèse valvulaire biologique ou artificielle (4, 5) destinée à l'utilisation dans l'organisme humain ou animal pour remplacer une valvule d'organe ou de vaisseau, en particulier prothèse valvulaire cardiaque ou veineuse, comprenant un stent (8) ou sans stent, avec une structure valvulaire de support, comprenant au moins une valvule (7) et au moins une boucle conductrice (2) qui constitue l'inductance d'un circuit oscillant résonnant électrique,
**caractérisée en ce que**
l'au moins une boucle conductrice (2) constitue la structure valvulaire propre et/ou la valvule propre (7) et a une fonction de support auprès de la prothèse valvulaire et **en ce que** la boucle conductrice (2) est revêtue d'un non conducteur en tant que couche d'isolation.

2. Prothèse valvulaire selon la revendication 1, **caractérisée en ce que** le stent (8) et/ou la structure valvulaire présentent du côté extérieur une pluralité de cellules de tailles identiques et/ou différentes.

3. Prothèse valvulaire selon la revendication 1 ou 2, **caractérisée en ce que** la prothèse valvulaire (4, 5) peut être pliée et déployée et est réalisée de manière à pouvoir ainsi être implantée par voie percutanée.

4. Prothèse valvulaire selon l'une quelconque des revendications précédentes, **caractérisée en ce que** des moyens d'ancrage, de préférence sous la forme d'oeillets, de crochets ou similaires, sont prévus au niveau de la boucle conductrice (2) et/ou au niveau de la structure valvulaire et **en ce que** de préférence, les moyens d'ancrage sont réalisés d'une seule pièce avec la boucle conductrice (2).

5. Prothèse valvulaire selon l'une quelconque des revendications précédentes, **caractérisée en ce que** les moyens d'ancrage, dans l'état comprimé, ne dépassent pas de la prothèse valvulaire (4, 5) et agissent seulement à partir du dépliement.

6. Prothèse valvulaire selon l'une quelconque des revendications précédentes, **caractérisée en ce qu'**une valvule cardiaque biologique sans stent (1), en particulier provenant d'un animal, par exemple d'un cochon (valvule cardiaque, péricarde) ou d'un boeuf (péricarde) ou de tissus et cellules humains, est entourée par au moins une boucle conductrice (2).

7. Prothèse valvulaire selon l'une quelconque des revendications précédentes, **caractérisée en ce que** des parties biorésorbables qui stabilisent la prothèse valvulaire (4, 5) pour l'implantation sont prévues, **en ce que** l'au moins une boucle conductrice (2) est prévue pour l'implantation dans une partie biologique de la prothèse valvulaire (4, 5) et reste à cet endroit après que les parties biorésorbables dans le corps ont été démontées.

8. Prothèse valvulaire selon l'une quelconque des revendications précédentes, **caractérisée en ce que** la structure valvulaire et la valvule (7) sont fixées au moins en partie par la boucle conductrice (2) dans un stent auto-dépliable ou expansible mécaniquement de telle sorte que lors de la compression et du dépliement subséquents, la structure valvulaire ainsi que la valvule (7) et la boucle conductrice (2) reviennent à la forme, la position et la géométrie souhaitées, l'étanchéité nécessaire de la prothèse dans le palier étant réalisée.

9. Prothèse valvulaire selon l'une quelconque des revendications précédentes, **caractérisée en ce que** la structure valvulaire est réalisée avec plusieurs circuits oscillants résonnants ayant chacun une boucle conductrice (2) ou **en ce que** plusieurs circuits oscillants résonnants sont accouplés les uns aux autres avec des fréquences de résonance différentes mais seulement une seule boucle conductrice (2).

10. Prothèse valvulaire selon l'une quelconque des revendications précédentes, **caractérisée en ce que** la structure valvulaire présente plusieurs formes de bobines différentes, en particulier réalisées sous forme de bobine cylindrique, de bobine en forme de selle ou de bobine en forme de cage à oiseau.

11. Prothèse valvulaire selon l'une quelconque des revendications précédentes, **caractérisée en ce qu'**au moins une bobine du circuit oscillant raisonnant présente des géométries pliables, des boucles conductrices croisées ou une structure en méandres et **en ce que** de préférence la piste conductrice de la boucle conductrice (2) est réalisée en forme de méandres.

12. Prothèse valvulaire selon l'une quelconque des revendications précédentes, **caractérisée en ce que** la boucle conductrice (2) présente la forme d'une valvule cardiaque biologique et **en ce qu'**au moins deux boucles de la boucle conductrice (2) sont prévues, lesquelles forment avec leurs faces une forme cylindrique qui entoure la forme naturelle de valvule en forme de tuyau souple et qui maintiennent les sorties des vaisseaux du coeur dans les régions entre les boucles.

13. Prothèse valvulaire selon l'une quelconque des revendications précédentes, **caractérisée en ce que** la capacité (6) appartenant au circuit oscillant résonnant est fixée/montée sur et/ou dans la prothèse valvulaire (4, 5) et/ou est intégrée dans la conception de la prothèse valvulaire (4, 5).

14. Prothèse valvulaire selon l'une quelconque des revendications précédentes, **caractérisée en ce que** l'inductance et/ou la capacité (6) du circuit oscillant sont prévues au moins en partie pour l'intégration dans le tissu enveloppant la prothèse valvulaire (4, 5), pour une couture, un ancrage et/ou une fermeture étanche.

15. Prothèse valvulaire selon l'une quelconque des revendications précédentes, **caractérisée en ce que** la boucle conductrice (2) est revêtue de plastique, tel que du téflon, du polyester, du polyuréthane, du parylène ou des polymères similaires et/ou de céramique et **en ce que** de préférence le revêtement présente une épaisseur d'au moins 1 nm, en particulier comprise entre 1 nm et 200 µm.

16. Prothèse valvulaire selon l'une quelconque des revendications précédentes, **caractérisée en ce qu'**une capacité interne est réalisée par la couche d'isolation et la distance entre les boucles des boucles conductrices (2).

17. Prothèse valvulaire selon l'une quelconque des revendications précédentes, **caractérisée en ce qu'**une capacité supplémentaire (6) du circuit oscillant est réalisée au moyen d'un condensateur.

18. Prothèse valvulaire selon l'une quelconque des revendications précédentes, **caractérisée en ce qu'**une bobine supplémentaire et/ou la capacité supplémentaire (6) sont fixées ou montées sur et/ou dans ou contre la structure valvulaire à l'aide de la couche d'isolation.

19. Prothèse valvulaire selon l'une quelconque des revendications précédentes, **caractérisée en ce que** la boucle conductrice (2) est fixée ou montée sur la structure valvulaire au moyen d'une couche.

20. Prothèse valvulaire selon l'une quelconque des revendications précédentes, **caractérisée en ce que** le circuit oscillant résonnant possède une fréquence de résonance, en particulier dans la plage de 8 à 400 MHz, qui correspond à la fréquence d'un champ magnétique extérieur, en particulier d'un tomographe à RM.

21. Prothèse valvulaire selon l'une quelconque des revendications précédentes, **caractérisée en ce que** la boucle conductrice (2) est découpée dans un fil ou dans un tube ou dans une tôle, et est fabriquée en un matériau ayant une susceptibilité magnétique faible et/ou une bonne conductivité électrique.

22. Prothèse valvulaire selon l'une quelconque des revendications précédentes, **caractérisée en ce que** la boucle conductrice (2) présente un support de conducteur électriquement conducteur ou électriquement non conducteur qui est revêtu au moyen d'un matériau conducteur, notamment de l'or, du platine, du tantale, de l'argent ou du cuivre et/ou d'alliages conducteurs.

23. Prothèse valvulaire selon l'une quelconque des revendications précédentes, **caractérisée en ce que** la boucle conductrice (2) est réalisée par un revêtement de la prothèse valvulaire avec un matériau électriquement conducteur et **en ce que** notamment le matériau électriquement conducteur ou non conducteur de la prothèse valvulaire (4, 5) est vaporisé ou pulvérisé avec le matériau électriquement conducteur.

24. Prothèse valvulaire selon l'une quelconque des revendications précédentes, **caractérisée en ce que** notamment dans le cas de polymères, de céramique, de nickel-titane et de titane, en tant que matériau du support de conducteur, on applique sur la boucle conductrice (2) une couche de base afin d'améliorer l'adhésion d'un revêtement.

25. Prothèse valvulaire selon l'une quelconque des revendications précédentes, **caractérisée en ce que** la prothèse valvulaire (4, 5) est réalisée de manière pliable ou dépliable et de telle sorte que lors du dépliement, la fréquence de résonance soit modifiée par la variation de la géométrie du circuit oscillant de la bobine et puisse être ajustée à la fréquence de résonance du tomographe à RM, de sorte que le processus de dépliement lors de l'implantation puisse être suivi par le biais de la variation de la caractéristique du signal dans l'image RM.

26. Prothèse valvulaire (4, 5) biologique ou artificielle, expansible et pouvant être implantée par voie percutanée, en particulier selon l'une quelconque des revendications précédentes, destinée à l'utilisation dans l'organisme humain et/ou animal pour remplacer une valvule d'organe ou de vaisseau, comprenant au moins un mécanisme valvulaire pourvu d'une valvule (7) et comprenant un stent (8) présentant une douille en matériau biocompatible, le stent (8), dans la hauteur fonctionnelle de la valvule, étant entouré par au moins une boucle conductrice (2) qui constitue l'inductance d'un circuit oscillant résonnant électrique, **caractérisée en ce que** le stent (8) présente une structure porteuse et la structure porteuse est formée par la boucle conductrice (2) et la boucle conductrice (2) a une fonction de support auprès de la prothèse valvulaire.

27. Prothèse valvulaire selon la revendication 26, **caractérisée en ce que** le mécanisme valvulaire dans le stent (8) est fixé au moyen de la boucle conductrice (2) à la douille du stent (8), et **en ce que** de préférence, des régions de la boucle conductrice (2) sortant de la douille sont réalisées sous forme de moyens d'ancrage, en particulier sous forme de crochets et/ou d'oeillets.
